# EUROPÄISCHE PATENTSCHRIFT

(11) **EP 1 040 193 B1**
(45) Veröffentlichungstag und Bekanntmachung des Hinweises auf die Patenterteilung: **17.11.2004**
(21) Anmeldenummer: 98966671.4
(22) Anmeldetag: 23.12.1998
(51) Int. Cl.: C12N 15/52, C12N 15/53, C12P 25/00, C12N 9/02, C12N 15/80

(54) **PROMOTOR AUS ASHBYA GOSSYPII**
PROMOTER FROM ASHBYA GOSSYPII
PROMOTEUR ISSU D'ASHBYA GOSSYPII

(30) Priorität: 23.12.1997 DE 19757755
(43) Veröffentlichungstag der Anmeldung: 04.10.2000
(73) Patentinhaber: BASF AKTIENGESELLSCHAFT, 67056 Ludwigshafen (DE)
(72) Erfinder: REVUELTA DOVAL, Jose Luis, E-37001 Salamanca (ES); SANTOS GARCIA, Maria Angeles, E-37009 Salamanco (ES); POMPEJUS, Markus, D-67165 Waldsee (DE); SEULBERGER, Harald, D-67141 Neuhofen (DE)
(86) Internationale Anmeldenummer: PCT/EP1998/008439
(87) Internationale Veröffentlichungsnummer: WO 1999/033993

(56) Entgegenhaltungen:
- WO-A-92/00379

## Beschreibung

Die vorliegende Erfindung betrifft einen neuen Promotor aus Ashbya gossypii und dessen Verwendung zur Genexpression.

Filamentöse Pilze sind bedeutende Organismen für die Biotechnologie aufgrund ihrer Eigenschaft, interessante Inhaltsstoffe zu produzieren. Zu diesen von Pilzen gebildeten Inhaltsstoffen gehören sowohl niedermolekulare Verbindungen wie organische Säuren, Antibiotika oder Vitamine als auch höhermolekulare Verbindungen wie Enzyme, nicht-enzymatische Proteine oder andere Biopolymere.

Es ist ein allgemeines Ziel der Biotechnologie, die Produktion solcher Inhaltsstoffe durch die Pilze zu optimieren. Zu diesem Zweck werden unter anderem auch gentechnische Methoden verwendet. Dabei werden z.B. einzelne Gene oder Gruppen von Genen in dem jeweiligen filamentösen Pilz überexpremiert. Diese Gene können aus dem Organismus stammen in dem sie expremiert werden, aber auch aus anderen Organismen.

In allen Fällen ist es notwendig die Expression der zu exprimierenden Gene steuern zu können. Alle exprimierten Gene in allen Organismen besitzen 5' der kodierenden Sequenz eine Promotorregion. Diese Region ist für den Start der Transkription selbst verantwortlich als auch für die Regulation der Transkription. Diese Regulation findet in der Regel statt durch Bindung von Transkriptionsfaktoren an regulatorische Sequenzen innerhalb der Promotorregion. Promotoren sind in der Regel frei portierbar, d.h. man kann einen Promotor von einem Gen verwenden, um die Transkription eines anderen Gens zu steuern. Diese Steuerung des neuen Gens ist dann in der Regel identisch zur Steuerung der originären Gens von dem der Promotor stammt. Man kann somit mit einem bekannten Promotor, dessen Regulation man kennt und steuern kann, die Expression eines beliebigen Gens in bekannter Weise steuern.

Der filamentöse Pilz Ashbya gossypii ist von biotechnologischem und wissenschaftlichem Interesse. Insbesondere interessant ist er aufgrund seiner Eigenschaft große Mengen an Riboflavin (siehe z.B. Kurth et al. (1996) Riboflavin, in: Ullmann's Encyclopedia of industrial chemistry, VCH Weinheim) produzieren zu können. Daneben ist er aber auch interessant zur Produktion anderer Metabolite und Inhaltsstoffe. Diese Inhaltsstoffe können z.B.

Aminosäuren, Vitamine, Proteine, aber auch andere Stoffe des Primär- und Sekundärmetabolismus oder andere Biopolymere sein.

Zur Optimierung der Produktion solcher Inhaltsstoffe bei Ashbya gossypii ist, wie bei anderen Organismen, die Anwendung gentechnischer Methoden aussichtsreich.

Zur Realisierung solch eines Vorhabens sind Ashbya gossypii - eigene genetische Regulatoren (insbesondere Promotoren) von großer Bedeutung. Insbesondere sind solche (sogenannte starke) Promotoren interessant, die eine Überexpression von Genen in Ashbya gossypii ermöglichen. Solche starken Promotoren aus Ashbya sind bislang nicht beschrieben.

Lediglich das Dokument WO 9 200 379 beschreibt die Isolierung und Nutzung der Promotorregion des Translationselongationsfaktors EF-1α aus Ashbya gossypii.

Aufgabe der vorliegenden Erfindung war es, starke Regulationselemente der Transkription (Promotoren) bereitzustellen, die vor allem in filamentösen Pilzen, bevorzugt solchen der Gattung Ashbya, zur Anwendung kommen können.

Gegenstand der Erfindung ist eine als Promotor geeignete DNA-Sequenz enthaltend die in SEQ ID NO:1 Position 9 bis 307 dargestellte Primärstruktur.

Ein besonders bevorzugter Promotor ist die in SEQ ID NO:1 dargestellte DNA-Sequenz, die am 5'- und 3'-Ende eine jeweils 8 Nukleotide lange NotI Restriktionsschnittstelle trägt, die ein bequemes Portieren des Promotors erlaubt.

Für die Funktion als Promotor wichtige Merkmale sind:
- die bona fide TATA Box (nt 224-230),
- zwei Sequenzabschnitte (nt 43-51 und 77-85), die der Erkennungssequenz des sogenannten "GCR1 binding elements" entsprechen und
- einen Sequenzabschnitt, (nt 9-20) dessen Komplement partial der Erkennungssequenz des "RAP1 binding elements" entspricht.

Ein weiterer Gegenstand der Erfindung ist die Verwendung der erfindungsgemäßen Promocorsequenzen in Expressionskassetten, wobei die Promotorsequenzen funktionell mit einem oder mehreren Strukturgenen verknüpft werden. Als funktionelle Verknüpfung wird eine solche Anordnung von DNA-Sequenzen bezeichnet, die die Transkription des oder der Strukturgene gestatten.

Mit Hilfe solcher Expressionskassetten können Wirtsorganismen wie Bakterien, Hefen, Pilze, Tiere und Pflanzen transformiert werden. Bevorzugte Wirtsorganismen sind Hefen und Pilze, insbesondere filamentöse Pilze wie solche der Gattung Ashbya.

Ein weiterer Gegenstand der Erfindung ist ein Verfahren zur rekombinanten Herstellung von Feinchemikalien in Wirtsorganismen wobei
ein Wirtsorganismus mit einer der oben beschriebenen Expressionskassetten transformiert wird und diese Expressionskassette ein oder mehrere Strukturgene enthält, die für die gewünschte Feinchemikalie codieren oder die Biosynthese der gewünschten Feinchemikalie katalysieren,
der transformierte Wirtsorganismus gezüchtet wird und
die gewünschte Feinchemikalie aus dem Züchtungsmedium isoliert wird.

Dieses Verfahren ist für Feinchemikalien wie Enzyme, Vitamine, Aminosäuren, Zucker, Fettsäuren, natürliche und synthetische Geschmacks-, Aroma- und Farbstoffe breit anwendbar. Die Züchtung der transformierten Wirtsorganismen sowie die Isolierung aus den Wirtsorganismen bzw. aus dem Züchtungsmedium erfolgt mit dem Fachmann bekannten Verfahren.

Die weitere Ausgestaltung der Erfindung ist in den folgenden Beispielen beschrieben.

### Beispiel 1

### Herstellung einer genomischen Genbank aus Ashbya gossypii ATCC10895

Genomische DNA aus Ashbya gossypii ATCC10895 kann nach üblichen Verfahren präpariert werden, z.B. wie beschrieben in EP 9703208. Die genomische Genbank, ausgehend von dieser DNA, kann nach üblichen Methoden (z.B. Sambrook, J. et al. (1989) Molecular cloning: a laboratory manual, Cold Spring Harbor Laboratory Press oder Ausubel, F.M. et al. (1994) Current protocols in molecular biology, John Wiley and sons) in beliebigen Plasmiden oder Cosmiden, wie z.B. SuperCos1 (Stratagene, La Jolla, USA) erstellt werden.

### Beispiel 2

### Der GAP-Promotor aus Ashbya gossypii

Das Gen für Glycerinaldehyd-3-Phosphat-Dehydrogenase aus Ashbya gossypii (AgGAP) kann man durch ein allgemein übliches Screening einer genomischen Ashbya gossypii Cosmid-Genbank (siehe Beispiel 1, mit einer Sonde, die aus Sequenzinformationen des GAP Gens aus Saccharomyces cerevisiae erstellt wurde) klonieren.

Der 5' nicht-translatierte Bereich des Gens (-373 bis -8 Region, bezogen auf den Translationsstart) wurde als Promotor angenommen. Flankierend zu dieser Sequenz wurden 2 Schnittstellen für die Restriktionsendonuklease NotI eingeführt. In diesem Bereich findet man die bona fide TATA Box (nt 224-230), zwei Sequenzabschnitte (nt 43-51 und 77-85), die dem sogenannten GCR1 binding element und einen Sequenzabschnitt, (nt 9-20) dessen Komplement partial dem RAP1 binding element von Saccharomyces cerevisiae entspricht (siehe z.B. Johnston, M. und Carlson, M. (1992) pp.193-281 in The molecular biology and cellular biology of the yeast Saccharomyces: Gene expression, Cold Spring Harbor Laboratory Press).

Die so konstruierte Promotorkassette kann als einfach portierbares Expressionssignal vor jedes beliebige Gen gesetzt werden und führt zu deutlicher Überexpression des jeweiligen Gens in Ashbya gossypii, wie gezeigt in Beispiel 3. Die resultierende Sequenz ist die Sequenz der SEQ ID NO:1.

### Beispiel 3

### Konstruktion eines Konstruktes des AgADE4Gens mit dem GAP-Promotors aus Ashbya gossypii und Überexpression des AgADE4-Gens in Ashbya gossypii

Zur Einfügung der GAP-Promotorkassette 5' der kodierenden Region des AgADE4 wurde nach üblichen Methode (z.B. Glover, D.M. und Hames, B.D. (1995) DNA cloning Vol.1, IRL press) 8 bp 5' des ATG Startcodon eine singuläre NotI Schnittstelle (Erkennungssequenz GCGGCCGC) eingeführt.

Die GAP-Promotorkassette (Beispiel 2) kann dann über NotI in diese Position eingefügt werden. Analog kann man vorgehen bei der Klonierung der GAP-Promotorkassette 5' der kodierenden Region anderer Gene von Ashbya gossypii (wie z.B. die rib-Gene), aber auch heterologer Gene (d.h. die nicht aus Asbhya gosypii stammen, wie z.B. Gene für beliebige Enzyme).

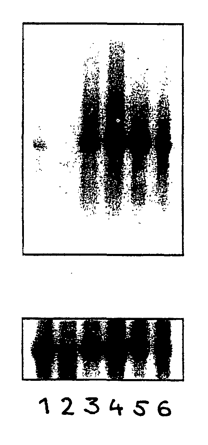

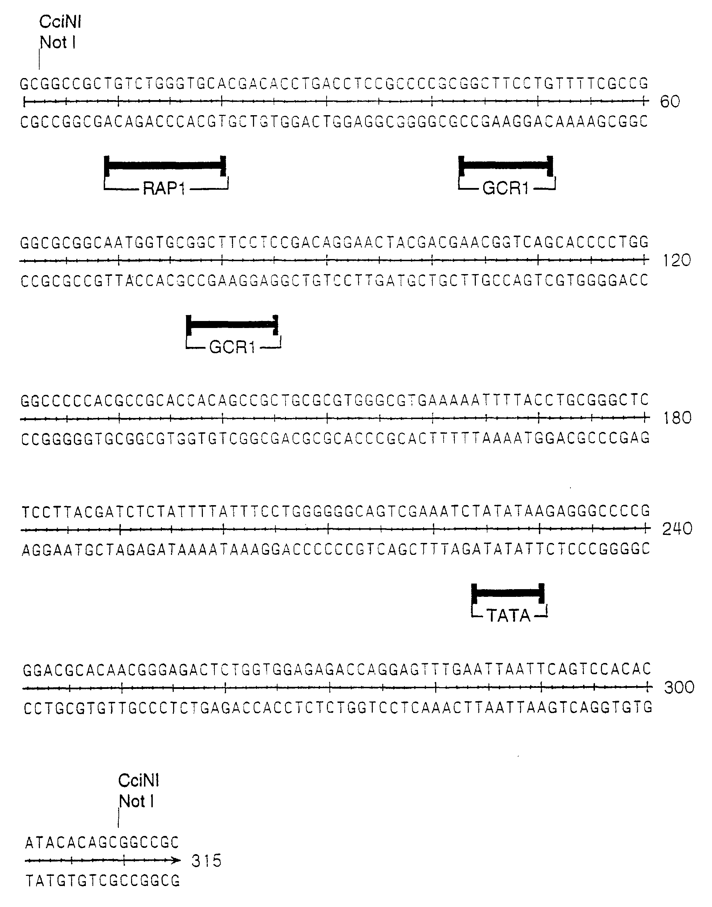

In Ashbya gossypii wird die Expression der Gene, die die GAP-Promotorkassette 5' der kodierenden Region tragen durch den GAP-Promotor kontrolliert. Exemplarisch ist dies dargestellt mit einem Northern blot in Abbildung 1.

### Beschreibung der Abbildung 1:

Northern Blot gesamter RNA aus Ashbya gossypii mit einem Fragment von AgADE4 (oberer Teil) bzw. als interne konstitutive Kontrolle mit dem AgGPD1 Gen (unterer Teil).

Spurenbelegung von links nach rechts:
1. RNA aus ASHBYA gossypii / Wild-Typ
2. RNA aus ASHBYA gossypii / ade4::G418
3. RNA aus ASHBYA gossypii / GAP-AgADE4
4. RNA aus ASHBYA gossypii / GAP-AgADE4
5. RNA aus ASHBYA gossypii / GAP-AgADE4-A418W
6. RNA aus ASHBYA gossypii / GAP-AgADE4-D310V-K333A

### (1) ALGEMEINE INFORMATION:

(i) ANMELDER:
   (A) NAME: BASF Aktiengesellschaft
   (B) STRASSE: Carl-Bosch-Strasse 38
   (C) ORT: Ludwigshafen
   (E) LAND: Bundesrepublik Deutschland
   (F) POSTLEITZAHL: D-67056
   (G) TELEPHON: 0621/6048526
   (H) TELEFAX: 0621/6043123
   (I) TELEX: 1762175170
(ii) ANMELDETITEL: GAP-Promotor aus Ashbya gossypii
(iii) ANZAHL DER SEQUENZEN: 1
(iv) COMPUTER-LESBARE FORM:
   (A) DATENTRÄGER: Floppy disk
   (B) COMPUTER: IBM PC compatible
   (C) BETRIEBSSYSTEM: PC-DOS/MS-DOS
   (D) SOFTWARE: PatentIn Release #1.0, Version #1.25 (EPA)

### (2) INFORMATION ZU SEQ ID NO: 1:

(i) SEQUENZ CHARAKTERISTIKA:
   (A) LÄNGE: 315 Basenpaare
   (B) ART: Nukleins"ure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Ashbya gossypii
(ix) MERKMALE:
   (A) NAME/SCHLÜSSEL: TATA_signal
   (B) LAGE: 224..230
(ix) MERKMALE:
   (A) NAME/SCHLÜSSEL: misc_signal
   (B) LAGE: 43..51
(ix) MERKMALE:
   (A) NAME/SCHLÜSSEL: misc_signal
   (B) LAGE: 77..85
(xi) SEQUENZBESCHREIBUNG: SEQ ID NO: 1:
   GCGGCCGCTG TCTGGGTGCA CGACACCTGA CCTCCGCCCC GCGGCTTCCT GTTTTCGCCG    60
   GGCGCGGCAA TGGTGCGGCT TCCTCCGACA GGAACTACGA CGAACGGTCA GCACCCCTGG   120
   GGCCCCCACG CCGCACCACA GCCGCTGCGC GTGGGCGTGA AAAATTTTAC CTGCGGGCTC   180
   TCCTTACGAT CTCTATTTTA TTTCCTGGGG GGCAGTCGAA ATCTATATAA GAGGGCCCCG   240
   GGACGCACAA CGGGAGACTC TGGTGGAGAG ACCAGGAGTT TGAATTAATT CAGTCCACAC   300
   ATACACAGCG GCCGC                                                    315

## Patentansprüche

1. Als Promotor geeignete DNA-Sequenz enthaltend
a) die in SEQ ID NO:1 Position 9 bis 307 dargestellte Primärstruktur.

2. DNA-Sequenz nach Anspruch 1 mit der in SEQ ID NO:1 Position 1-315 dargestellten Primärstruktur.

3. Expressionskassette enthaltend eine DNA-Sequenz nach Anspruch 1 oder 2 funktionell verknüpft mit einem Strukturgen und ggf. weiteren regulatorischen DNA-Sequenzen.

4. Verfahren zur rekombinanten Herstellung von Feinchemikalien in Wirtsorganismen wobei
ein Wirtsorganismus mit einer Expressionskassette nach Anspruch 3 transformiert wird und diese Expressionskassette ein oder mehrere Strukturgene enthält, die für die gewünschte Feinchemikalie codieren oder die Biosynthese der gewünschten Feinchemikalie katalysieren,
der transformierte Wirtsorganismus gezüchtet wird und
die gewünschte Feinchemikalie aus dem Züchtungsmedium isoliert wird.

5. Verwendung einer DNA-Sequenz nach Anspruch 1 oder 2 zur Genexpression in prokaryontischen oder eukaryontischen Wirtsorganismen.

6. Verwendung nach Anspruch 5 zur Genexpression in filamentösen Pilzen.

7. Verwendung nach Anspruch 6 zur Genexpression von homologen und heterologen Genen in Ashbya gossypii.

8. Verwendung nach Anspruch 5 zur Expression von Genen des Purinstoffwechsels.

## Claims

1. A DNA sequence which is suitable as promoter, containing
a) the primary structure shown in SEQ ID NO:1 position 9 to 307.

2. The DNA sequence as claimed in claim 1 with the primary structure shown in SEQ ID NO:1 position 1-315.

3. An expression cassette containing a DNA sequence as claimed in claim 1 or 2, functionally linked to a structural gene and, if appropriate, other regulatory DNA sequences.

4. A method for the recombinant production of fine chemicals in host organisms, where
a host organism is transformed with an expression cassette as claimed in claim 3 and this expression cassette contains one or more structural genes which encode the desired fine chemical or catalyze the biosynthesis of the desired fine chemical,
the transformed host organism is cultured and
the desired fine chemical is isolated from the culture medium.

5. The use of a DNA sequence as claimed in claim 1 or 2 for expressing genes in prokaryotic or eukaryotic host organisms.

6. The use as claimed in claim 5 for expressing genes in filamentous fungi.

7. The use as claimed in claim 6 for expressing homologous and heterologous genes in Ashbya gossypii.

8. The use as claimed in claim 5 for expressing genes of the purine metabolism.

## Revendications

1. Séquence d'ADN convenant comme promoteur, contenant
a) la structure primaire représentée dans SEQ ID NO:1 position 9 à 307.

2. Séquence d'ADN selon la revendication 1, avec la structure primaire représentée dans SEQ ID NO:1 position 1-315.

3. Cassette d'expression contenant une séquence d'ADN selon la revendication 1 ou 2, liée fonctionnellement à un gène structural et éventuellement d'autres séquences d'ADN régulatrices.

4. Procédé pour la préparation recombinante de produits de chimie fine dans des organismes hôtes, dans lequel
un micro-organisme hôte ayant une cassette d'expression selon la revendication 3 est transformé et cette cassette d'expression contient un ou plusieurs gènes structuraux qui codent pour les produits de chimie fine souhaités ou catalysent la biosynthèse des produits de chimie fine souhaités,
l'organisme hôte transformé est cultivé et le produit de chimie fine souhaité est isolé à partir du milieu de culture.

5. Utilisation d'une séquence d'ADN selon la revendication 1 ou 2 pour l'expression génique dans des micro-organismes hôtes procaryotes ou eucaryotes.

6. Utilisation selon la revendication 5 pour l'expression génique dans des champignons filamenteux.

7. Utilisation selon la revendication 6 pour l'expression génique de gènes homologues et hétérologues dans Ashbya gossypii.

8. Utilisation selon la revendication 5 pour l'expression de gènes du métabolisme de la purine.
